# EUROPÄISCHE PATENTSCHRIFT

(11) **EP 2 092 873 B1**
(45) Veröffentlichungstag und Bekanntmachung des Hinweises auf die Patenterteilung: **24.10.2012**
(21) Anmeldenummer: 09001283.2
(22) Anmeldetag: 30.01.2009
(51) Int. Cl.: A61B 1/00, A61B 1/005, A61B 1/267

(54) **Endoskop**
Endoscope
Endoscope

(30) Priorität: 19.02.2008 DE 102008009912
(43) Veröffentlichungstag der Anmeldung: 26.08.2009
(73) Patentinhaber: Karl Storz GmbH & Co. KG, 78532 Tuttlingen (DE)
(72) Erfinder: Efinger, Andreas, 78604 Rietheim (DE); Eisele, Hans-Peter, 78532 Tuttlingen (DE)
(74) Vertreter: Hofmeister, Frank

(56) Entgegenhaltungen:
- EP-A- 1 834 575
- DE-A1- 10 314 288
- DE-A1- 19 523 959
- DE-A1- 19 822 167
- DE-U1- 20 015 447
- US-A- 4 747 661

## Beschreibung

Die Erfindung betrifft ein Endoskop mit einem hohlen Schaft zur Aufnahme eines Bildleiters, wobei das distale Ende des Schaftes gegenüber der Längsachse des Schaftes abgewinkelt ausgebildet ist und der Schaft distalseitig über ein mit einer Durchgangsbohrung für den Bildleiter versehenes Endstück verschlossen ist.

Derartige als starre Endoskope ausgebildete Endoskope, deren distale Spitze gegenüber der Längsachse des Schaftes abgewinkelt ausgebildet ist, werden beispielsweise als Sinuskope zur Untersuchung von Nasennebenhöhlen und Stirnhöhlen verwendet. Die distale Abwinklung des Instrumentenschaftes ist durch die medizinische Anwendung bedingt.

Nachteilig bei derartigen aus der Praxis bekannten Endoskopen mit abgewinkelter distaler Schaftspitze ist, dass diese entweder bei ausreichender Bildgröße und Bildqualität der durch den Bildleiter erzeugten Bilder zu Reinigungszwecken nicht autoklavierbar sind, da es bei den hohen Temperaturen zu Brüchen des Bildleiters kommt, oder aber bei der Verwendung flexibler Bildleiter oder von Bildleitern mit kleinem Durchmesser diese Endoskope zwar autoklavierbar sind, jedoch hinsichtlich der Bildgröße und/oder Bildqualität zu wünschen übrig lassen.

Aus der DE 103 14 288 A1 ist ein derartiges Endoskop mit einem flexiblen Bildleiter bekannt, das aus einem mehrlagig aufgebauten Sondenschlauch besteht, in dem der flexibler Bildleiter gelagert ist. Am distalen Ende des Sondenschlauchs ist der Bildleiter mit einem Objektiv verklebt. Im Bereich der 180° Umlenkung des Sondenschlauchs verläuft der Bild leiter parallel zur Mittelachse des Sondenschlauchs.

Davon ausgehend liegt der Erfindung die Aufgabe zugrunde, ein Endoskop der eingangs genannten Art zu schaffen, dass bei ausreichender Bildgröße und Bildqualität des Bildübertragungssystems zu Reinigungszwecken autoklavierbar ist.

Die Lösung dieser Aufgabenstellung ist erfindungsgemäß dadurch gekennzeichnet, dass der Bildleiter so im Schaft angeordnet ist, dass der Biegeradius des Bildleiters im Bereich der Abwinklung des Schaftes größer ist als der Biegeradius des Schaftes im Bereich der Abwinklung und, dass die Durchgangsbohrung für den Bildleiter im Endstück schräg zur Mittelachse des Schaftes ausgebildet ist

Durch die nicht achsparallele Anordnung des Bildleiters innerhalb des Endoskopschaftes ist es möglich den Bildleiter so innerhalb des Endoskopschaftes anzuordnen, dass Biegeradius des Bildleiters im Bereich der Abwinklung des Schaftes größer ist als der Biegeradius des Schaftes im Bereich der Abwinklung und somit auch die am Bildleiter auftretenden Biegespannungen gegenüber der aus der Praxis bekannten achsparallelen Anordnung des Bildleiters so deutlich reduziert werden können, dass ein erfindungsgemäß ausgebildetes Endoskop auch bei der Verwendung von Bildleitern, die eine ausreichende Bildgröße und hohe Bildqualität gewährleisten, ohne die Bildleiter zu gefährden autoklavierbar ist.

Um dem Bildleiter auch im Bereich seiner Lagerung im distalen Endstück des Schaftes die Weiterführung des großen Biegeradius zu ermöglichen, ist die Durchgangsbohrung für den Bildleiter im Endstück schräg zur Mittelachse des Schaftes ausgebildet ist.

Gemäß einer bevorzugten Ausführungsform der Erfindung wird vorgeschlagen, dass der Bildleiter als semiflexibler Bildleiter ausgebildet ist, um auch bei einem relativ großen Bildleiterquerschnitt der Schaftbiegung folgen zu können.

Zum Abdichten und als Schutz des Bildleiters wird weiterhin vorgeschlagen, dass der Bildleiter innerhalb des Schaftes in einem, vorzugsweise als Metallrohr ausgebildeten, Führungsrohr gelagert ist.

Das Beobachten des Untersuchungsgebiets erfolgt über ein am distalen Ende des Bildleiters angeordnetes, aus mindestens einer Linse bestehenden Objektiv, das zum Ausgleich der Schieflage des Bildleiters im Bereich der distalseitigen Durchgangsbohrung vorteilhafterweise als Schrägblickoptik ausgebildet ist. Die Schrägblickoptik hat darüber hinaus den Vorteil, dass die Blickrichtung über die Längsachsrichtung hinaus erweiterbar ist, um beispielsweise auch weiter in proximaler Richtung gelegene Bereiche des Untersuchungsgebietes sichtbar zu machen.

Um einerseits den Bildleiter im Führungsrohr zu fixieren und andererseits zu gewährleisten, dass die am Bildleiter auftretenden Biegespannungen nicht auf die Objektivlinsen übertragen werden, wird mit der Erfindung vorgeschlagen, dass der Bildleiter und das Führungsrohr im Bereich des distalen Endes des Bildleiters miteinander verklebt sind, wobei der Bildleiter im Klebebereich vorteilhafterweise nicht gebogen ist, um die Biegespannungen in diesem Abschnitt abbauen zu können.

Weiterhin wird mit der Erfindung vorgeschlagen, dass der Bildleiter und das Objektiv miteinander verklebt sind.

Schließlich wird mit der Erfindung vorgeschlagen, dass die distalseitige Abwinklung des Schaftes gegenüber der Längsachse des Schaftes vorzugsweise 60° beträgt. Dieses Maß der Abwinklung des Endoskopschaftes hat sich bei der Ausbildung des Endoskops als Sinuskop zur Untersuchung der Nasennebenhöhlen und Stirnhöhlen als besonders geeignet erwiesen. Selbstverständlich sind auch stärkere und schwächere Abwinklungen im Rahmen der erfindungsgemäßen Ausbildung des Endoskops möglich.

Weitere Merkmale und Vorteile der Erfindung ergeben sich anhand der zugehörigen Zeichnung, in der ein Ausführungsbeispiel eines erfindungsgemäßen Endoskops nur beispielhaft dargestellt ist, ohne die Erfindung auf dieses Ausführungsbeispiel zu beschränken. In der Zeichnung zeigt:
- Fig. 1: eine Seitenansicht eines erfindungsgemäßen Endoskops;
- Fig. 2: eine vergrößerte Schnittdarstellung des Details II gemäß Fig. 1 und
- Fig. 3: eine vergrößerte Ansicht des Details III gemäß Fig. 2.

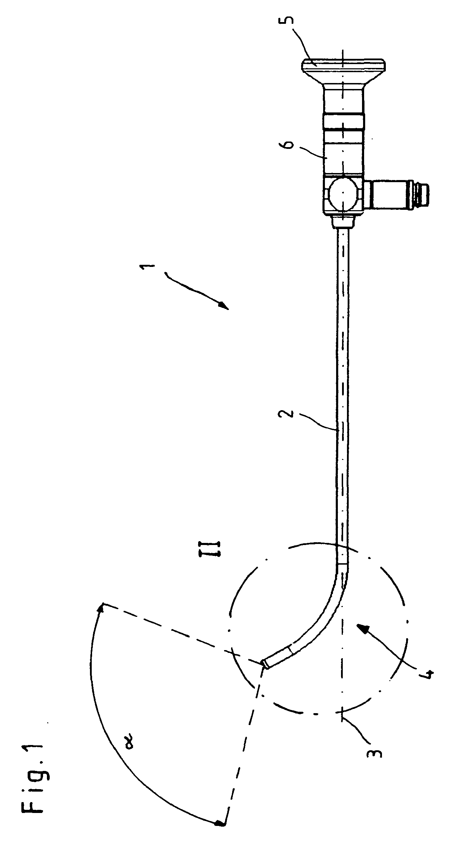

Das in den Abbildungen Fig. 1 bis 3 dargestellte Endoskop 1 besteht im wesentlichen aus einem hohlen Schaft 2, dessen distales Ende gegenüber der Längsachse 3 des Schaftes 2 eine seitliche Abwinklung 4 bildend abgewinkelt ausgebildet ist. Proximalseitig weist das Endoskop 1 eine mit einer Okulareinheit 5 versehene Handhabe 6 auf.

Bei dem dargestellten Endoskop 1 handelt es sich um ein Sinuskop zur Untersuchung der Nasennebenhöhlen und Stirnhöhlen. Die Abwinklung 4 des Schaftes 2 gegenüber der Längsachse 3 des Schaftes 2 ist medizinisch anatomisch bedingt, um das Endoskop 1 in das Untersuchungsgebiet einführen zu können. Vorteilhafterweise beträgt die Abwinklung gegenüber der Längsachse 3 60°, jedoch sind auch andere Winkelstellungen der Abwinklung 4 ausbildbar.

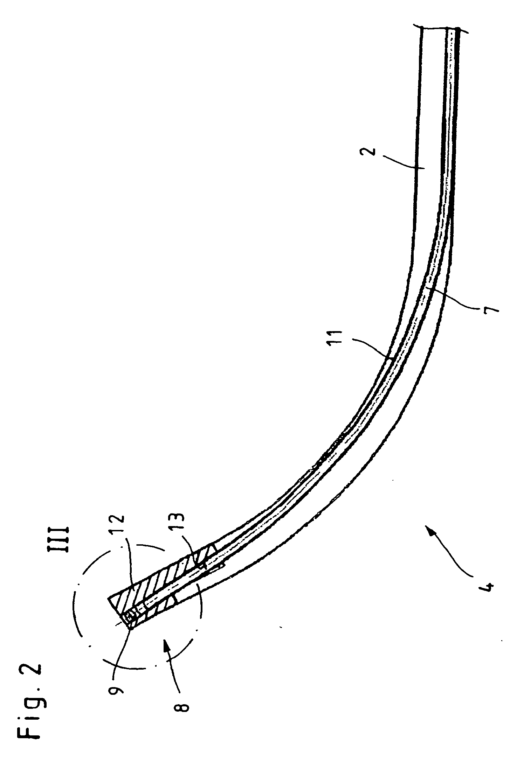

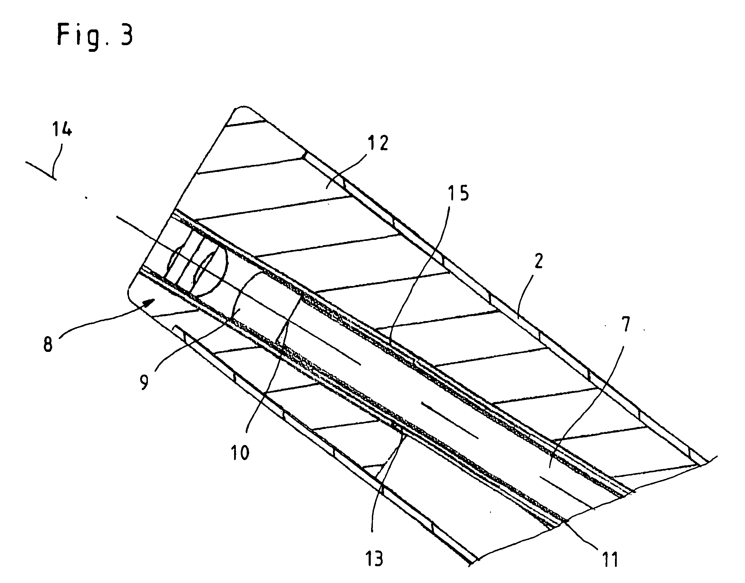

Wie aus den Schnittdarstellungen gemäß Fig. 2 und 3 ersichtlich, ist im Inneren des Schaftes 2 ein Bildleiter 7 angeordnet, der die Okulareinheit 5 zum Zwecke der Bildübertragung mit einem das distale Ende des Bildleiters 7 bildenden Objektiv 8 verbindet. Das Objektiv 8 seinerseits besteht bei der dargestellten Ausführungsform aus mehreren einzelnen Linsen 9, wobei das proximale Ende des Objektivs 8 und das distale Ende des Bildleiters 7 an einer Anlagefläche 10 miteinander verklebt sind. Bei dem Bildleiter 7 handelt um einen semiflexiblen Bildleiter 7, um auch bei einem relativ großen Bildleiterquerschnitt der Abwinklung 4 des Schaftes 2 folgen zu können.

Die Verwendung flexibler Bildleiter 7 ist nachteilig, da diese eine gegenüber den semiflexiblen Bildleitern 7 deutlich verringerte Bildqualität erzeugen.

Zum Abdichten und als Schutz des Bildleiters 7 ist der Bildleiter 7 innerhalb des Schaftes 2 in einem, vorzugsweise als Metallrohr ausgebildeten, Führungsrohr 11 gelagert.

Distalseitig ist der Schaft 2 über ein Endstück 12 verschlossen, welches mit einer Durchgangsbohrung 13 zur lagernden Aufnahme des Bildleiters 7 bzw. des mit dem Bildleiter 7 bestückten Führungsrohres 11 versehen ist.

Wie aus Fig. 2 ersichtlich, ist der Bildleiter 7 insbesondere im Bereich der Abwinklung 4 nicht mittig oder sonst wie achsparallel zur Mittelachse 14 des Schaftes 2 angeordnet. Die Lagerung des Bildleiters 7 im Schaft 2 ist vielmehr so ausgelegt, dass der Biegeradius des Bildleiters 7 im Bereich der Abwinklung 4 des Schaftes 2 größer ist als der Biegeradius des Schaftes 2 im Bereich der Abwinklung 4. Dieser größere Biegeradius auf Seiten des Bildleiters 7 bewirkt eine deutliche Reduzierung der auf den Bildleiter ausgeübten Biegespannungen im Vergleich zu den aus dem Stand der Technik bekannten Endoskopen, bei denen der Bildleiter achsparallel im Schaft 2 gelagert ist.

Durch diese Reduzierung der Biegespannung im Bildleiter 7 ist es möglich das Endoskop 1 zu Reinigungszwecken zu autoklavieren, wobei das Endoskop einer Temperatur von über 120°C ausgesetzt wird, ohne eine Beschädigung des Bildleiters 7, insbesondere im Bereich der Verklebung mit dem Objektiv 8, befürchten zu müssen.

Die biegespannungsarme Verlegung des Bildleiters 7 innerhalb des Schaftes 2 ermöglicht neben der Autoklavierbarkeit des Endoskops 1 die Verwendung semiflexibler Bildleiter 7 mit einem relativ großen Leiterquerschnitt, so dass auch die erhaltene Bildgröße für den Operateur vorteilhaft ist.

Der von der Achsparallelität abweichende Verlauf des Bildleiters 7 innerhalb des Schaftes 2 mit dem im Vergleich zum Schaft 2 größeren Biegeradius wird bei der dargestellten Ausführungsform dadurch bewirkt, dass die Durchgangsbohrung 13 für den Bildleiter 7 im Endstück 12 schräg zur Mittelachse 14 des Schaftes 2 ausgebildet ist, wie dies den Abbildungen Fig. 2 und 3 zu entnehmen ist.

Zum Ausgleich der Schieflage des Bildleiters 7 im Bereich der distalseitigen Durchgangsbohrung 13 ist die Objektivoptik vorteilhafterweise als Schrägblickoptik ausgebildet ist. Die Schrägblickoptik hat darüber hinaus den Vorteil, dass die Blickrichtung über die Längsachsrichtung hinaus erweiterbar ist, um beispielsweise auch weiter in proximaler Richtung gelegene Bereiche des Untersuchungsgebietes sichtbar zu machen. Ein gebräuchlicher Blickwinkel α des Objektivs 8 ist in Fig. 1 mit über 90° beispielhaft dargestellt.

Um einerseits den Bildleiter 7 im Führungsrohr 11 zu fixieren und andererseits zu gewährleisten, dass die am Bildleiter 7 auftretenden Biegespannungen nicht auf die Objektivlinsen 9 übertragen werden, sind der Bildleiter 7 und das Führungsrohr 11 im Bereich des distalen Endes des Bildleiters 7 miteinander verklebt, wobei der Bildleiter 7 im Klebebereich 15 vorteilhafterweise nicht gebogen, also gerade ausgebildet ist, um die Biegespannungen der vorherigen Biegung in diesem Abschnitt abbauen zu können.

Ein solchermaßen ausgebildetes Endoskop 1 zeichnet sich dadurch aus, dass es aufgrund der biegespannungsarmen Verlegung des Bildleiters 7 innerhalb des Schaftes 2 möglich ist, ein Endoskop 1 zu schaffen, das bei ausreichender Bildgröße und Bildqualität des Bildübertragungssystems zu Reinigungszwecken autoklavierbar ist.

### Bezugszeichenliste

- 1: Endoskop
- 2: Schaft
- 3: Längsachse
- 4: Abwinklung
- 5: Okulareinheit
- 6: Handhabe
- 7: Bildleiter
- 8: Objektiv
- 9: Linse
- 10: Anlagefläche/Klebestelle
- 11: Führungsrohr
- 12: Endstück
- 13: Durchgangsbohrung
- 14: Mittelachse
- 15: Klebebereich

- α: Blickwinkel

## Patentansprüche

1. Endoskop mit einem hohlen Schaft (2) zur Aufnahme eines Bildleiters (7), wobei das distale Ende des Schaftes (2) gegenüber der Längsachse (3) des Schaftes (2) abgewinkelt ausgebildet ist und der Schaft (2) distalseitig über ein mit einer Durchgangsbohrung (13) für den Bildleiter (7) versehenes Endstück (12) verschlossen ist,
**dadurch gekennzeichnet,**
**dass** der Bildleiter (7) so im Schaft (2) angeordnet ist, dass der Biegeradius des Bildleiters (7) im Bereich der Abwinklung (4) des Schaftes (2) größer ist als der Biegeradius des Schaftes (2) im Bereich der Abwinklung (4) und, dass die Durchgangsbohrung (13) für den Bildleiter (7) im Endstück (12) schräg zur Mittelachse (14) des Schaftes (2) ausgebildet ist.

2. Endoskop nach Anspruch 1, **dadurch gekennzeichnet, dass** der Bildleiter (7) als semiflexibler Bildleiter (7) ausgebildet ist.

3. Endoskop nach Anspruch 1 oder 2, **dadurch gekennzeichnet, dass** der Bildleiter (7) innerhalb des Schaftes (2) in einem Führungsrohr (11) gelagert ist.

4. Endoskop nach Anspruch 3, **dadurch gekennzeichnet, dass** das Führungsrohr (11) als Metallrohr ausgebildet ist.

5. Endoskop nach einem der Ansprüche 1 bis 4, **dadurch gekennzeichnet, dass** am distalen Ende des Bildleiters (7) ein aus mindestens einer Linse (9) bestehendes Objektiv (8) angeordnet ist.

6. Endoskop nach Anspruch 5, **dadurch gekennzeichnet, dass** das Objektiv (8) als Schrägblickoptik ausgebildet ist.

7. Endoskop nach einem der Ansprüche 3 bis 6, **dadurch gekennzeichnet, dass** der Bildleiter (7) und das Führungsrohr (11) im Bereich des distalen Endes des Bildleiters (7) miteinander verklebt sind.

8. Endoskop nach Anspruch 5, **dadurch gekennzeichnet, dass** der Bildleiter (7) im Verklebungsbereich mit dem Führungsrohr (11) ungebogen ausgebildet ist.

9. Endoskop nach einem der Ansprüche 5 bis 8, **dadurch gekennzeichnet, dass** der Bildleiter (7) und das Objektiv (8) miteinander verklebt sind.

10. Endoskop nach einem der Ansprüche 1 bis 9, **dadurch gekennzeichnet, dass** die distalseitige Abwinklung des Schaftes (2) gegenüber der Längsachse (3) des Schaftes (2), vorzugsweise 60°, beträgt.

## Claims

1. An endoscope with a hollow shaft (2) for accommodating an image guide (7), wherein the distal end of the shaft (2) is embodied in an angled manner with respect to the longitudinal axis (3) of the shaft (2) and the shaft (2) is closed on the distal side by means of an end piece (12) provided with a through bore (13) for the image guide (7), **characterized in that** the image guide (7) is arranged in the shaft (2) such that the bending radius of the image guide (7) in the region of the angle (4) of the shaft (2) is larger than the bending radius of the shaft (2) in the region of the angle (4) and that the through bore (13) for the image guide (7) in the end piece (12) is embodied in a slanting manner to the center axis (14) of the shaft (2).

2. The endoscope according to claim 1, **characterized in that** the image guide (7) is embodied as a semi-flexible image guide (7).

3. The endoscope according to claim 1 or 2, **characterized in that** the image guide (7) is supported in a guide tube (11) inside the shaft (2).

4. The endoscope according to claim 3, **characterized in that** the guide tube (11) is embodied as a metal tube.

5. The endoscope according to one of claims 1 through 4, **characterized in that** a lens system (8) comprising at least one lens (9) is arranged on the distal end of the image guide (7).

6. The endoscope according to claim 5, **characterized in that** the lens system (8) is embodied as an oblique-view optical system.

7. The endoscope according to one of claims 3 through 6, **characterized in that** the image guide (7) and the guide tube (11) are adhered to one another in the region of the distal end of the image guide (7).

8. The endoscope according to claim 5, **characterized in that** in the adhesion region the image guide (7) is embodied with the guide tube (11) without bending.

9. The endoscope according to one of claims 5 through 8, **characterized in that** the image guide (7) and the lens system (8) are adhered to one another.

10. The endoscope according to one of claims 1 through 9, **characterized in that** the distal-side angle of the shaft (2) is preferably 60° with respect to the longitudinal axis (3) of the shaft (2).

## Revendications

1. Endoscope avec une tige (2) creuse pour la réception d'un faisceau de fibres optiques (7), l'extrémité distale de la tige (2) étant prévue coudée par rapport à l'axe longitudinal (3) de la tige (2), et la tige (2) étant obturée du côté distal par une pièce d'extrémité (12) pourvue d'un alésage traversant (13) pour le faisceau de fibres optiques ('1),
**caractérisé**
**en ce que** le faisceau de fibres optiques (7) est disposé dans la tige (2) de telle manière que le rayon de courbure du faisceau de fibres optiques (7) dans la zone du coude (4) de la tige (2) est supérieur au rayon de courbure de la tige (2) dans la zone du coude (4), et en ce que l'alésage traversant (13) pour le faisceau de fibres optiques (7) dans la pièce d'extrémité (12) est prévu incliné par rapport à l'axe central (14) de la tige (2).

2. Endoscope selon la revendication 1, **caractérisé en ce que** le faisceau de fibres optiques (7) est réalisé comme faisceau de fibres optiques (7) semi-flexible.

3. Endoscope selon la revendication 1 ou la revendication 2, **caractérisé en ce que** le faisceau de fibres optiques (7) est logé dans un tube de guidage (11) à l'intérieur de la tige (2).

4. Endoscope selon la revendication 3, **caractérisé en ce que** le tube de guidage (11) est réalisé comme tube métallique.

5. Endoscope selon l'une des revendications 1 à 4, **caractérisé en ce qu'**un objectif (8) constitué d'au moins une lentille (9) est disposé à l'extrémité distale du faisceau de fibres optiques (7).

6. Endoscope selon la revendication 5, **caractérisé en ce que** l'objectif (8) est réalisé comme optique à visée oblique.

7. Endoscope selon l'une des revendications 3 à 6, **caractérisé en ce que** le faisceau de fibres optiques (7) et le tube de guidage (11) sont collés l'un à l'autre dans la zone de l'extrémité distale du faisceau de fibres optiques (7).

8. Endoscope selon la revendication 5, **caractérisé en ce que** le faisceau de fibres optiques (7) est prévu non plié dans la zone de collage avec le tube de guidage (11).

9. Endoscope selon l'une des revendications 5 à 8, **caractérisé en ce que** le faisceau de fibres optiques (7) et l'objectif (8) sont collés l'un à l'autre.

10. Endoscope selon l'une des revendications 1 à 9, **caractérisé en ce que** le coudage côté distal de la tige (2) est préférentiellement de 60° par rapport à l'axe longitudinal (3) de la tige (2).
